(19) 

(11) **EP 4 129 262 B1**

(12) **EUROPEAN PATENT SPECIFICATION**

(45) Date of publication and mention of the grant of the patent:
**16.09.2026 Bulletin 2026/38**

(21) Application number: **21778769.6**

(22) Date of filing: **01.02.2021**

(51) International Patent Classification (IPC):
***A61H 3/00*** *(2006.01)* ***A61F 5/01*** *(2006.01)*

(52) Cooperative Patent Classification (CPC):
**A61F 5/0113**

(86) International application number:
**PCT/JP2021/003534**

(87) International publication number:
**WO 2021/199648 (07.10.2021 Gazette 2021/40)**

(54) **LOWER-LIMB ORTHOTIC DEVICE**

ORTHESEVORRICHTUNG FÜR DIE UNTEREN GLIEDMASSEN

DISPOSITIF ORTHOPÉDIQUE DE MEMBRE INFÉRIEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.03.2020 JP 2020061721**

(43) Date of publication of application:
**08.02.2023 Bulletin 2023/06**

(73) Proprietor: **Toray Industries, Inc.**
**Tokyo 103-8666 (JP)**

(72) Inventors:
- **CHEN, Yao**
  **Otsu-shi, Shiga 520-2141 (JP)**
- **YAMADA, Satoshi**
  **Otsu-shi, Shiga 520-2141 (JP)**
- **SUZUKI, Hidetoshi**
  **Otsu-shi, Shiga 520-2141 (JP)**
- **OHATA, Koji**
  **Kyoto-shi, Kyoto 606-8501 (JP)**

(74) Representative: **Mewburn Ellis LLP**
**Aurora Building**
**Counterslip**
**Bristol BS1 6BX (GB)**

(56) References cited:
**WO-A1-2015/133479**
**WO-A1-2019/159281**
**GB-A- 2 375 962**
**IE-A1- 20 100 556**
**JP-A- 2005 028 025**
**US-A- 5 257 969**
**US-A- 5 860 423**
**US-A1- 2003 204 157**
**US-A1- 2015 320 581**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 129 262 B1

## Description

TECHNICAL FIELD

The present invention relates to a lower-limb orthotic device.

BACKGROUND ART

Conventionally, various lower-limb orthotic devices have been proposed for preventing deformation such as equinus foot and drop foot, function improvement, and daily operation improvement for a person who cannot freely move muscles and joints of the lower limb due to functional deterioration or loss of the lower limb due to disorders such as stroke hemiplegia and infantile paralysis, or other disorders. The lower-limb orthotic devices are mainly hard holding tools such as metal and plastic, and are excellent in holding force and orthotic force to an affected part.

In addition, a lower-limb orthotic device using a flexible material has been proposed, and for example, Patent Document 1 has disclosed a foot orthotic capable of lifting the tip of the foot upward without blocking the operation of the foot. Specifically, the technique includes a first attachment to be attached to a foot of a person, a second attachment to be attached to a thigh of the person, and an elastic material such as a rubber band to be attached so as to be stretched between the first attachment and the second attachment. When the elastic material is attached so as to extend, an elastic force of the elastic material acts to bring the first attachment and the second attachment close to each other. As a result, the foot is elastically urged toward the front side of the lower leg, and the tip of the foot is lifted upward. In addition, there has been disclosed a technique in which the urging force allows operation of the foot in a certain degree, and in a case where the function of lowering the tip of the foot is not impaired, the tip of the foot can be lowered by itself against the urging force. Patent Documents 2 to 5 disclose further orthopedic devices.

PRIOR ART DOCUMENT

PATENT DOCUMENT

Patent Document 1: JP H9-313553;
Patent Document 2: US 5257969 A;
Patent Document 3: US 2015/320581 A1;
Patent Document 4: GB 2375962 A;
Patent Document 5: IE 20100556 A1;
Patent Document 6: US 5860423 A

SUMMARY OF THE INVENTION

PROBLEMS TO BE SOLVED BY THE INVENTION

However, a fixed type lower-limb orthotic device using a hard or semi-hard material such as metal or plastic causes physical and mental pain of a patient, such as discomfort caused by contact with the skin when the lower-limb orthotic device is worn, pain caused by hitting of a hard part in body operation, poor appearance, a need of an increase in size of the shoe of the foot on which the lower-limb orthotic device is worn so that shoes of the same size on the left and right cannot be worn, and the like. Caregivers have also pointed out poor detachability. When a person walks while wearing the lower-limb orthotic device at home, a portion of a hard material comes into contact with a floor, an inner wall, or the like and is rubbed against the floor, the inner wall, or the like, so that the lower-limb orthotic device is damaged such as being damaged on both sides. Therefore, many problems such as not wearing the lower-limb orthotic device indoors have been pointed out.

The lower-limb orthotic device described in Patent Document 1 works by bringing the two attachments close to each other with the elastic material, and is problematic in that it takes time and effort to wear the two attachments and to mount the elastic material. The elastic material is made of an elastically stretchable material represented by a rubber band, and the biasing force thereof only lifts the tip of the foot upward, so that it is not possible to guide and hold the foot portion to the original normal position, and there is a problem that a desired orthotic effect or function improvement effect cannot be obtained.

An object of the present invention is to provide an orthotic device that can improve the problems of the prior art, can achieve both a holding force and an orthotic force necessary for improvement of deformation and function of a lower

limb and the trackability to friction, fluctuation, compression and the like occurring during operation between an orthotic site and the orthotic device is easy to wear, does not impair an appearance in wearing, maintains an appropriate holding force and orthotic force for a long period of time, and thus can be continuously used.

## SOLUTIONS TO THE PROBLEMS

In order to achieve the object, the present invention has the following configurations.

A lower-limb orthotic device including: a fabric band A1 covering at least a part of an ankle portion including a malleolus and/or at least a part of a knee portion; a fabric band A2 covering a foot portion including a metatarsal bone; and at least one fabric band B, in which at least a part of the fabric band B covers the foot portion including a fifth metatarsal bone or a cuboid bone, the fabric band B is disposed to be positioned from the foot portion including the fifth metatarsal bone or the cuboid bone to the ankle portion and/or the knee portion, one end of the fabric band B in a longitudinal direction is connected to the fabric band A1, the other end of the fabric band B is connected to the fabric band A2, an interior angle between the fabric band A1 and the fabric band B when the lower-limb orthotic device is worn is 30° to 90°, and at least one of the fabric bands has an extension ratio at a load of 20 N/cm (100 N/5 cm) of 0.5% or more and 50% or less in a width direction and a longitudinal direction or in either one of the width direction and the longitudinal direction, wherein polyester-based thermoplastic elastomer fibers are contained as fibers constituting the fabric band A1, the fabric band A2, and the fabric band B.

In some embodiments of the lower-limb orthotic device, the fabric band B includes a length adjustment mechanism.

In some embodiments of the lower-limb orthotic device, the one or more kinds of thermoplastic elastomer fibers contained as fibers constituting the fabric band A1, the fabric band A2, and the fabric band B include one or more kinds selected from polyurethane elastic fibers and thermoplastic polyester elastomer fibers.

In some embodiments of the lower-limb orthotic device, polyester-based thermoplastic elastomer fibers are contained as fibers constituting the fabric band A1, the fabric band A2, and the fabric band B.

In some embodiments, the lower-limb orthotic device further includes one or more fabric bands C, in which at least a part of the fabric band C is disposed to cover the foot portion including a first metatarsal bone, and the fabric band C is formed by connecting one end of the fabric band C in a longitudinal direction to the fabric band A1 and the other end of the fabric band C to the fabric band A2 so as to be positioned from the foot portion including the first metatarsal bone to the ankle portion and/or the knee portion.

In some embodiments of the lower-limb orthotic device, an interior angle between the fabric band B and the fabric band C is 30° to 80°.

In some embodiments of the lower-limb orthotic device, one or more kinds of thermoplastic elastomer fibers are contained as fibers constituting the fabric band C.

In some embodiments of the lower-limb orthotic device, polyester-based thermoplastic elastomer fibers are contained as fibers constituting the fabric band C.

In some embodiments of the lower-limb orthotic device, when the extension ratios of the fabric bands A1, A2, B, and C at a load of 20 N/cm (100 N/5 cm) are represented as $A1_E$, $A2_E$, $B_E$, and $C_E$, respectively, $A1_E \geq C_E \geq B_E$ and $A2_E \geq C_E \geq B_E$ are satisfied.

In some embodiments of the lower-limb orthotic device, at least one of the fabric bands has an extension ratio at a load of 4 N/cm (20 N/5 cm) of 0.1% or more and 10% or less in the width direction and the longitudinal direction or in either one of the width direction and the longitudinal direction.

In some embodiments of the lower-limb orthotic device described, the lower-limb orthotic device is formed integrally with a leg wear that covers at least a knee to the foot portion, and includes a mechanism that is openable and closable during wearing.

In some embodiments, the lower-limb orthotic device is a brace that does not include a metal or hard resin frame, or a monocoque.

## EFFECTS OF THE INVENTION

According to the present invention, it is possible to provide an orthotic device that achieves both a holding force and an orthotic force necessary for improvement of deformation and function of a lower limb and the trackability to friction, fluctuation, compression and the like occurring during operation between an orthotic site and the orthotic device, is easy to wear, does not impair appearance during wearing, and in addition, maintains an appropriate holding force and orthotic force for a long period of time, and can be continuously used.

The lower-limb orthotic device according to the present invention can be preferably used for orthoses such as a lower limb orthosis for treatment and rehabilitation for the purpose of preventing deformation of the foot portion, improving the function, and improving the operation of daily life; however, the application range is not limited thereto.

BRIEF DESCRIPTION OF THE DRAWINGS

Fig. 1 is a conceptual diagram illustrating an embodiment in which respective fabric bands are connected in the present invention.
Fig. 2 is an enlarged view of a portion indicated by a broken line ellipse in Fig. 1.
Fig. 3 is a conceptual diagram illustrating a lower-limb orthotic device using a fabric band C.
Fig. 4 is an enlarged view of a portion indicated by a broken line ellipse in Fig. 3.
Fig. 5 is a conceptual diagram illustrating an embodiment in which a fabric band B includes a length adjustment mechanism.

EMBODIMENTS OF THE INVENTION

A lower-limb orthotic device according to the present invention includes a fabric band A1 covering at least a part of an ankle portion including a malleolus and/or at least a part of a knee portion, a fabric band A2 covering a foot portion including a metatarsal bone, and at least one fabric band B, in which at least a part of the fabric band B covers the foot portion including a fifth metatarsal bone or a cuboid bone, and the fabric band B is disposed to be positioned from the foot portion including the fifth metatarsal bone or the cuboid bone to the ankle portion and/or the knee portion.

It is necessary that at least one of the fabric bands used for the lower-limb orthotic device of the present invention has an extension ratio at a load of 20 N/cm (100 N/5 cm) of 0.5% or more and 50% or less in a width direction and a longitudinal direction or in either one of these directions. It is preferable that at least one of the fabric bands has an extension ratio at a load of 20 N/cm (100 N/5 cm) of 1% or more and 20% or less in the width direction and the longitudinal direction or in either one of these directions. In other words, it is preferable that the extension ratio of the fabric band at a load of 20 N/cm (100 N/5 cm) is 1% or more and 20% or less in warp and weft directions or in either one of these directions. It is more preferable that the above range is satisfied in all of the fabric bands A1, A2, and B, and, in the case of using the fabric band C, the fabric band C as well.

In the case of the fabric band in which the extension ratio at a load of 20 N/cm (100 N/5 cm) satisfies the above range in both the warp direction and the weft direction, either one of the warp and weft directions may be regarded as a longitudinal direction (or a width direction) of the fabric band. When only one of the warp direction and the weft direction satisfies the above range, it is preferable to use a direction satisfying the above range as the longitudinal direction. In particular, it is preferable that the weft direction of the woven fabric used for the fabric band is designed to satisfy the above range, and the weft direction is set to the longitudinal direction from the viewpoint of obtaining stable quality.

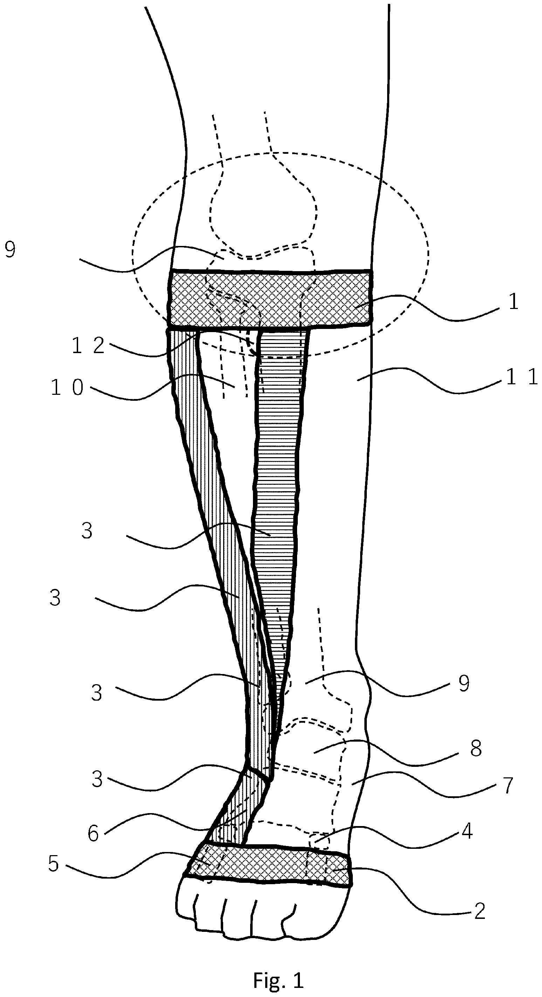

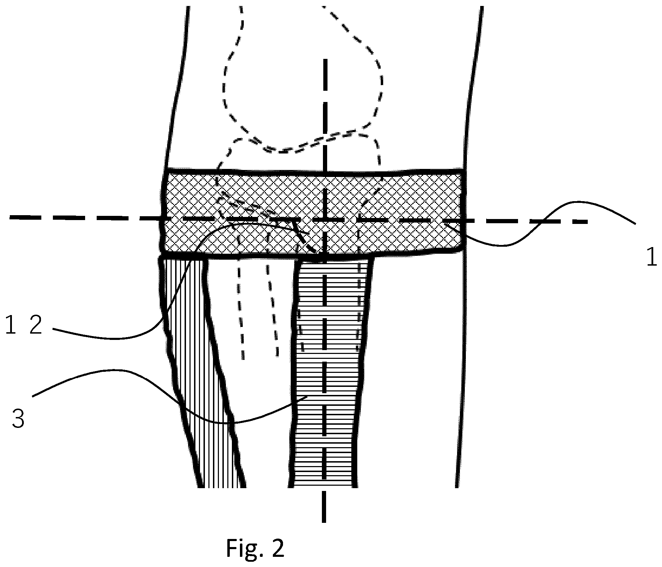

Fig. 1 is a conceptual diagram illustrating an embodiment in which respective fabric bands are connected in the present invention, and Fig. 2 is an enlarged view of a portion indicated by a broken line ellipse in Fig. 1. Note that Fig. 1 has been described by taking the right foot as an example, but the same applies to the left foot. In the present invention, a case where simply "fabric band" is described without any particular description, such as "fabric band A1", indicates any one of the fabric bands A1, A2, and B (any one of fabric bands A1, A2, B, and C when the fabric band C described below is included).

The fabric band A1 (1) and the fabric band A2 (2) are connected to the fabric band B (3) and have a function as a fulcrum for maintaining an orthotic force by the fabric band B (3) without reducing the orthotic force such as shift, tear, and excessive extension, with respect to a desired orthotic force by the fabric band B (3) when the lower-limb orthotic device is worn.

The fabric band A1 (1) covers at least a part of the ankle portion including the malleolus and/or at least a part of the knee portion, and usually surrounds at least a part of the ankle portion including the malleolus and/or at least a part of the knee portion in a circling manner. The malleolus in the case of the ankle portion including the malleolus and the central portion having the maximum diameter of the calf in the case of the knee portion are caught by the downward push of the fabric band A1 (1) during wearing to prevent the downward push of the fabric band A1 (1), and the fabric band A1 (1) functions as a fulcrum, so that the orthotic force is stabilized. Note that, the knee portion described herein includes the upper calf, the popliteal fossa, and the upper knee, and covering at least a part of the knee may be surrounding to cover at least a part of the knee portion in a circling manner. The malleolus refers to a foot portion including a tibia (9) and a central to lower end portion of a fibula (10), and the ankle portion refers to a foot portion including at least a part of a talus (8) and a calcaneus (7). Covering at least a part of the ankle portion including the malleolus means that the fabric band may surround at least a part of the malleolus and/or the ankle portion in a circling manner.

It is preferable that at least one end of the fabric band A1 (1) is configured to be openable and closable, and the size and the wearing pressure can be finely adjusted.

The fabric band A2 (2) covers the foot portion including the metatarsal bone, and usually goes around the foot portion including the metatarsal bone. It is preferable that at least one end of the fabric band A2 (2) is configured to be

openable and closable, and the size and the wearing pressure can be finely adjusted.

The wearing pressure of each of the fabric bands A1 and A2 with respect to the human body when the lower-limb orthotic device is worn is preferably 1 hPa to 20 Pa and more preferably 1 hPa to 15 Pa. By setting the wearing pressure of the fabric bands A1 and A2 with respect to the human body when the lower-limb orthotic device is worn in the above range, congestion due to the inhibition of blood flow by a high wearing pressure does not occur, and the lower-limb orthotic device can be comfortably worn.

At least a part of fabric band B (3) is disposed to cover a fifth metatarsal bone (5) or a cuboid bone (6) and is positioned from the foot portion including the fifth metatarsal bone (5) or the cuboid bone (6) to the ankle portion and/or the knee portion. The fabric band B may be connected to the fabric band A2 (2) in the vicinity of the fifth metatarsal bone (5), or may be extended from the cuboid bone (6) toward a first metatarsal bone (4) via the sole to cover the metatarsal bone on the sole side of the foot, and connected to the fabric band A2 (2) in the vicinity of the metatarsal bone on the sole side in the middle thereof.

The number of the fabric bands B may be one or more.

In the lower-limb orthotic device of the present invention, in order to guide the ankle joint in a desired direction while developing a desired orthotic force by the fabric band B during wearing, the interior angle of a connecting portion between the fabric band B and the fabric band A1 during wearing is 30° to 90° and preferably 40° to 80°.

An interior angle (12) between the fabric band A1 and the fabric band B is an angle formed by a center line of the fabric band A1 (1) and a center line of the fabric band B (3) when the lower-limb orthotic device is worn on one foot requiring correction, and refers to an angle of 90° or less among angles formed by center lines. The center line is indicated by a broken line in Fig. 2. When the interior angle is smaller than 30°, a desired orthotic force by the fabric band B during wearing cannot be exhibited, and the ankle joint requiring correction cannot be guided in a desired direction, so that the orthotic function as the lower-limb orthotic device is unsuitable.

In the above description, the ankle joint refers to a joint of an ankle, and is a joint in which the tibia (9), the fibula (10), and the talus (8) are combined and reinforced by ligaments present in the joint, and which is responsible for a motion to deflect the ankle up and down or to the right and left.

The equinus foot is a kind of deformation of the ankle joint, and is a symptom in which the ankle is extended in a manner of standing on the tip of the foot, does not return freely, and the heel does not touch the floor or hardly touches the floor during standing or walking. Regardless of the presence or absence of deformation of the ankle joint itself, due to the contracture of the Achilles tendon and the contracture of the muscles present in the lower limbs such as the triceps surae muscle, a force for directing the tip of the foot downward is generated, and the foot may not return to a normal position or may exhibit a symptom of difficulty in returning. At this time, the ankle joint is further inverted, and is also in a so-called inversion state in many cases. This is a symptom that is called a talipes equinovarus.

The drop foot is a symptom in which the function of bending the foot back is lost due to the decrease in the muscles present in the lower limbs, and the tip of the foot remains directed downward. When the symptom of the drop foot continues for a long period of time as it is, the symptom may be fixed, resulting in a symptom of the equinus foot.

When these symptoms of the equinus foot and the drop foot are shown, inconvenience occurs in daily life, for example, the heel does not touch or hardly touches the ground in the standing position, the foot cannot touch the ground from the heel or hardly touches the ground when the foot separate from the ground is caused to touch the ground at the time of walking, the foot touches the ground from the toe side or the outside of the foot portion, or walking is performed while rubbing the toe during walking.

In response to such a symptom, by guiding the ankle joint in a desired direction, it is possible to improve the symptom by correcting the state and movement of the foot portion so as to approximate to normal movement as much as possible with respect to the difficulty of movement that causes the deformation of the foot portion such as the equinus foot and the drop foot, and the functional deterioration in function of the foot portion such as walking or the like. Although there is no symptom of the equinus foot and the drop foot, when there is an inconvenience in the ankle joint due to illness or the like, the symptom in the ankle joint may be improved by performing the correction in the similar manner.

For example, the talipes equinovarus, which is one of typical examples of the equinus foot, is a symptom in which the tip of the foot is directed inward (so-called inversion) due to the contracture of the Achilles tendon and the muscles present in the lower limbs such as the triceps surae muscle as described above, or due to abnormal muscle tension or the like. In the present invention, when the lower-limb orthotic device is worn, at least a part of the lower-limb orthotic device is disposed to cover the fifth metatarsal bone or the cuboid bone, and the fabric band B is appropriately disposed to be positioned from the foot portion including the fifth metatarsal bone or the cuboid bone to the ankle portion and/or the knee portion. Therefore, the orthotic force for guiding the foot portion to the original normal position is exerted when the tip of the foot is directed inward. This orthotic force is particularly useful for the talipes equinovarus, but of course, is also effective against instability of other ankle portions.

The method of connecting of the fabric band B and the fabric band A can be appropriately selected as long as an orthotic force in a desired direction is satisfied. Specifically, sewing with a sewing machine or the like, a snap button, a hook-and-loop fastener, a buckle, a loop, and the like are used.

The extension ratio of the fabric band B used in the present invention at a load of 10 N/cm (50 N/5 cm) in the longitudinal direction is preferably 0.5% to 15%. Accordingly, it is possible to easily apply a desired orthotic force. The extension ratio is more preferably 0.8% to 12%.

An extension change rate of the fabric band B at a load of 10 N/cm (50 N/5 cm) in the longitudinal direction is preferably 20% or less, further preferably 15% or less, and more preferably 10% or less after extension and restoration of the fabric band B at a load of 10 N/cm (50 N/5 cm) in the longitudinal direction are repeated 1000 times. The lower limit is preferably smaller, and specifically preferably 0% or more.

When the extension ratios of the fabric bands (the fabric bands A1, A2, B, and C) at a load of 20 N/cm (100 N/5 cm) are represented as $A1_E$, $A2_E$, and $B_E$ respectively, $A1_E \geq B_E$ and $A2_E \geq B_E$ are preferably satisfied. It is preferable to satisfy $A1_E = A2_E = B_E$ since it is efficient in that the fabric bands can be produced with the same fabric. In this case, "=" does not need to be strictly the same, and $A1_E \geq B_E$ and $A2_E \geq B_E$ are maintained, but $A1_E$ and A2E may each be within approximately 10% of $B_E$.

By setting the extension ratio in the above range, the force to return to the original orthotic position moderately acts on the force applied by the foot portion of the wearer when the wearer wears this lower-limb orthotic device and moves, whereby an appropriate orthotic position is maintained, which is effective for function recovery and daily operation improvement. In addition, even when the wearer puts on the lower-limb orthotic device of the present invention and then operates for a long period of time, the lower-limb orthotic device is less likely to lose its shape, can exhibit its initial performance continuously for a long period of time, can be continuously used by maintaining an appropriate holding force and orthotic force for a long period of time, and exhibits more effects on function recovery and daily operation improvement.

In the present invention, the fabric band C other than the fabric band A and the fabric band B may be disposed. One or more of the fabric bands C can be used. It is preferable that at least a part of the fabric band C is disposed to cover the first metatarsal bone, and one end of the fabric band C in the longitudinal direction is connected to the fabric band A1 and the other end is connected to the fabric band A2 so that the fabric band C is positioned from the foot portion including the first metatarsal bone to the ankle portion and/or the knee portion. From the viewpoint of exerting a higher effect, an aspect is preferable in which the fabric band A1 is disposed to cover at least a part of the knee portion, and the fabric band C is disposed to be positioned over the knee portion and is connected to the fabric band A1.

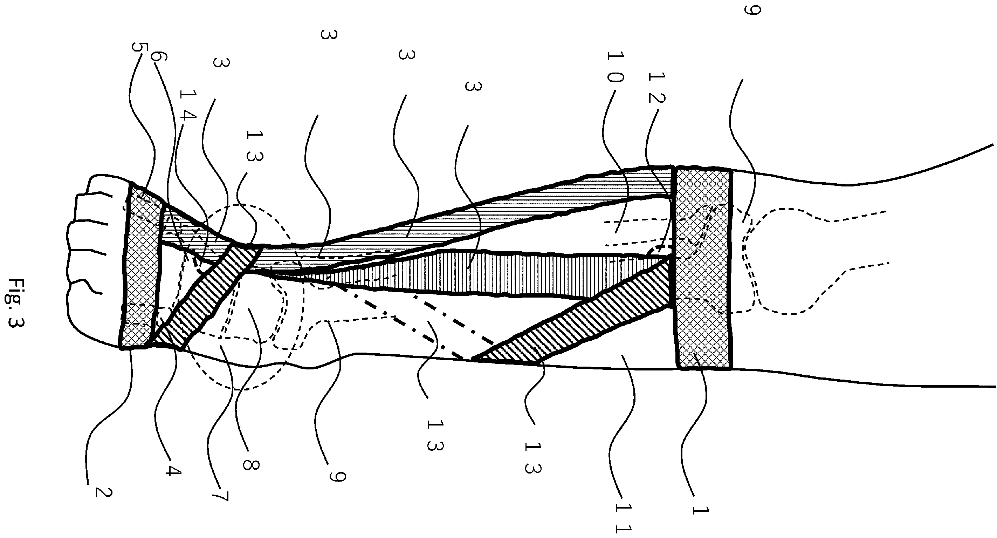

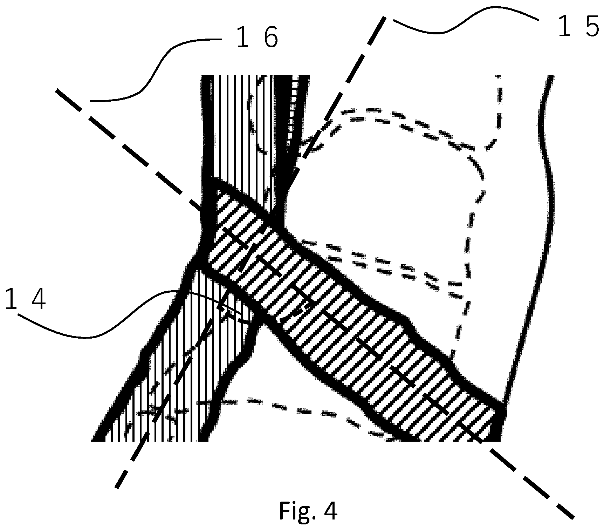

Fig. 3 is a conceptual diagram illustrating a lower-limb orthotic device using the fabric band C, and Fig. 4 is an enlarged view of a portion indicated by a broken line ellipse in Fig. 3. Note that, the drawings illustrate the right foot, but the same applies to the left foot. Hereinafter, description will be made will reference to this point.

The fabric band C (13) may be disposed to cover the foot portion including the first metatarsal bone (4) and cover the ankle portion including the Achilles tendon from the outside to the inside from the lateral portion over from the foot portion to the instep as illustrated in Fig. 3, or may be disposed to cover the ankle portion including the Achilles tendon from the outside to the inside from the lateral portion over from the foot portion to the instep and cover the knee portion from the lower leg (11) including the tibia (9). Although not illustrated in Fig. 3, as other methods, the fabric band C (13) may be disposed to cover the foot portion including the first metatarsal bone (4) and cover the medial side of the ankle portion including the Achilles tendon from the lateral side of the calcaneus (7) over from the plantar medial side to the plantar lateral side, or may be disposed to cover the foot portion including the first metatarsal bone (4), cover the medial side of the ankle portion including the Achilles tendon from the lateral side of the calcaneus (7) over from the plantar medial side to the plantar lateral side, and cover the knee portion from the lower leg (11) including the tibia (9). By disposing the fabric band C (13) in this manner and setting an interior angle described below in an appropriate range, it is possible to suppress excessive inward bending of the front half of the foot portion including the metatarsal bone. In particular, the latter disposition is more preferable in that the foot is easy to touch the ground from the ball of the foot (thumb).

In this case, when the extension ratios of fabric bands (the fabric bands A1, A2, B, and C) at a load of 20 N/cm (100 N/5 cm) are represented as $A1_E$, $A2_E$, $B_E$, and $C_E$, respectively, $A1_E \geq C_E \geq B_E$ and $A2_E \geq C_E \geq B_E$ are preferably satisfied. It is preferable to satisfy $A1_E = A2_E = C_E = B_E$ since it is efficient in that the fabric bands can be produced with the same fabric. In this case, "=" does not need to be strictly the same, and $A1_E \geq C_E \geq B_E$ and $A2_E \geq C_E \geq B_E$ are maintained, but A1E, A2E, and $C_E$ may each be within approximately 10% of $B_E$.

When the fabric bands are connected in this way, the fabric band C usually intersects with the fabric band B, partially overlaps with the fabric band B, or is in contact with the fabric band B (this portion is collectively referred to as a "contacting portion").

In the case of using the fabric band C, the interior angle (14) between the fabric band C (13) and the fabric band B (3) when the lower-limb orthotic device is worn is preferably 30° to 80° and more preferably 35° to 70°.

An interior angle (14) between the fabric band B and the fabric band C is an angle of 90° or less among angles formed by center lines of each of the fabric band B (3) and the fabric band C (13) when the lower-limb orthotic device is worn on one foot requiring correction. The center line is indicated by a broken line as the center line (15) of the fabric band B and the center line (16) of the fabric band C in Fig. 4. When the interior angle is 30° or more, a desired orthotic force by the fabric band C during wearing can be exhibited efficiently, and the ankle joint requiring correction is easily guided in a desired direction, so that the orthotic function as the lower-limb orthotic device is excellent.

By disposing the fabric band C and the fabric band B so that the interior angle of a connecting portion between the fabric band B and the fabric band C in the above range when the lower-limb orthotic device is worn, in a case where the foot portion of the wearer is unstable during operation and the front half of the foot portion including the metatarsal bone excessively bends inward, or in a case where the orthotic force for suppressing the inversion of the fabric band B excessively acts and the front half of the foot portion excessively bends inward, an appropriate orthotic position is maintained, which can be more effective for function recovery and daily operation improvement.

As described above, the fabric band C can be used by connecting the end thereof in the longitudinal direction to each of the fabric band A1 and the fabric band A2, and can be further connected to the fabric band B. The connection in this case can be usually performed at a portion where the fabric bands B and C are in contact. Thereby, the risk that the interior angle of the connecting portion between the fabric band C and the fabric band B deviates from the desired range can be reduced, and the ease of wearing is further improved, which is preferable.

The method of connecting the fabric band C, the fabric band A1, the fabric band A2, and the fabric band B can be appropriately selected as long as a desired orthotic effect when the lower-limb orthotic device is worn is satisfied. Specifically, there is used sewing with a sewing machine or the like, a hook-and-loop fastener, a snap button, or the like.

The weave structure of the fabric band is not particularly limited as long as the range defined in the present invention is satisfied; however, at least one or more structures such as plain weave, twill weave, satin weave, and double weave in combination of these structures can be appropriately selected according to the application.

It is preferable that the woven fabric used for the fabric band has elastic property, and the extension ratio at a load of 4 N/cm (20 N/5 cm) is 0.1% to 10% in the warp direction and the weft direction or in either one of these directions. It is preferable that at least one of the fabric bands satisfies this range, and it is more preferable that all of the fabric bands satisfy the above range.

The extension ratio of the woven fabric used for the fabric band at a load of 10 N/cm (50 N/5 cm) is preferably 0.5% to 15% in the warp and weft directions or in either one of these directions in any one of the fabric bands A1, A2, B, and C. It is preferable that at least one of the fabric bands satisfies this range, and it is more preferable that all of the fabric bands satisfy the above range.

In the case of the woven fabric in which the extension ratio at a load of 10 N/cm (50 N/5 cm) satisfies the above range in both the warp direction and the weft direction as the fabric band, either one of the warp and weft directions may be regarded as a longitudinal direction (or a width direction) of the fabric band. When only one of the warp direction and the weft direction satisfies the above range, it is preferable to use a direction satisfying the above range as the longitudinal direction. In particular, it is preferable that the weft direction of the woven fabric is designed to satisfy the above range, and thus the weft direction can be set to the longitudinal direction from the viewpoint of obtaining stable quality.

By setting the extension ratio in the above range, since the fabric band is extended according to the strength of operation, the fabric band is appropriately extended without excessively fixing the foot portion, the friction between the foot portion and the lower-limb orthotic device is reduced, the trackability to fluctuation of the foot portion during operation, the compression on the region covered by the fabric band that occurs when the orthotic force acts, and the like is further improved, and the pain and discomfort during wearing are easily suppressed, which is preferable. In addition, when the fabric band receives the force applied by the wearer during operation, the muscles are easily moved, and function recovery and daily operation improvement are effectively obtained, which is preferable. When the motion of this lower-limb orthotic device during wearing is within a certain stress range, the fabric band is appropriately extended and exerts an orthotic force to the foot portion while following the motion, and when the motion exceeds the certain stress range, the fabric band stably fixes the foot portion without following the motion and guides the foot portion into an appropriate orthotic range.

The form of a yarn to be used for the fabric band may be either a short fiber or a long fiber as long as the range defined in the present invention is satisfied. The material of the yarn is not limited to natural fibers, synthetic fibers, and the like as long as the range defined in the present invention is satisfied, and various fibers can be used.

Specifically, there can be appropriately used synthetic fibers such as polyester fibers, polyamide fibers, and thermoplastic elastomer fibers, cellulose fibers such as rayon and cotton, and natural fibers such as wool and silk. As the yarn, these fibers may be used alone or in combination of two or more.

In order to impart elastic property, an elastic fiber that is a stretchable material is combined with the above yarn. As the elastic fiber to be mixed, at least one or more thermoplastic elastomer fibers are used.

As the thermoplastic elastomer fibers, specifically, polyurethane elastic fibers, thermoplastic polyester elastomer fibers, and the like can be used, and from the viewpoint of easily obtaining dimensional stability and elastic property with high elasticity, thermoplastic polyester elastomer fibers are more preferred.

The fineness of the fiber used for the fabric band is preferably 30 decitex to 1500 decitex, and more preferably 50 decitex to 1000 decitex.

When the above elastic fibers and other fibers (inelastic fibers) are used, the fineness of the elastic fibers is preferably 50 decitex to 1500 decitex, and more preferably 156 decitex to 1000 decitex. The fineness of the inelastic fibers as other fibers is preferably 30 decitex to 1000 decitex, and more preferably 50 decitex to 500 decitex.

It is preferable to use an elastic fiber as a part of the yarn to be used as described above, but specifically, a

configuration is adopted such that an elastic fiber is used for any one of the warp and the weft or at least a part of the both, thereby allowing appropriate adjustment of: the dimensional stability of the woven fabric; the elastic property of the elastic fiber and a good recoverability after extension, the holding force, orthotic force, or dimensional stability to guide the ankle joint in a desired direction; and the trackability to the operation.

In the above case, the use ratio of the elastic fibers to be used is appropriately determined within a range in which desired properties are obtained, and is preferably about 30 wt% to 70 wt% in the warp or weft, or in the entire woven fabric.

When other fibers (inelastic yarns) that are fibers other than elastic fibers are used in the fabric band, the extension ratio can be adjusted by appropriately adjusting the crimp ratio. When an inelastic fiber is used for either the warp or the weft and the crimp ratio thereof is set to 5% or more and 30% or less, desired extensibility can be obtained. The crimp ratio is preferably 10% or more and 30% or less.

When elastic fibers and inelastic fibers are used for either the warp or the weft, the crimp ratio of the inelastic fibers is set to preferably 5% or more and 30% or less. As a result, when the elastic fibers are extended, the inelastic fibers follow the crimp ratio, and excessive extension by the elastic fibers can be stopped.

The crimp ratio of the elastic fiber in this case is also appropriately set according to desired properties, and is preferably 0% or more and 5% or less.

The weave density of the fabric band used in the present invention is preferably 7.87 yarns/cm (20 yarns/inch (2.54 cm)) to 59.06 yarns/cm (150 yarns/inch (2.54 cm)), more preferably 11.81 yarns/cm (30 yarns/inch (2.54 cm)) to 39.37 yarns.cm (100 yarns/inch (2.54 cm)) in warp density and weft density, respectively.

The extension recovery rate of the woven fabric used for the fabric band after extension is repeated 50 times at a load of 20 N/cm (100 N/5 cm) is preferably 80% to 100%, further preferably 85% to 100%, and more preferably 90% to 100% in the width direction and the longitudinal direction or in either one of these directions. It is preferable that at least one of the fabric bands satisfies this range, and it is more preferable that all of the fabric bands satisfy the above range.

Preferably, the extension recovery rate after extension is repeated 50 times is set within the above range and the force to return to the original orthotic position moderately acts on the force applied by the foot portion of the wearer during operation, whereby an appropriate orthotic position is maintained, which is effective for function recovery and daily operation improvement. In addition, the appropriate holding force and the orthotic force are maintained for a long period of time, allowing the lower-limb orthotic device of the present invention to be continuously used, which are more effective for function recovery and daily operation improvement.

A most preferable fabric band in the present invention is a plain woven fabric composed of inelastic fibers with one of the warp and the weft having a crimp ratio of 10% or more and 30% or less and elastic fibers with at least a part of the other having a crimp ratio of 0% or more and 5% or less. The weave density is preferably 11.81 yarns/cm (30 yarns/inch (2.54 cm)) to 39.37 yarns/cm (100 yarns/inch (2.54 cm)) in warp density and weft density.

The fineness is preferably 50 decitex to 500 decitex for inelastic fibers and 156 decitex to 1000 decitex for elastic fibers.

The lower-limb orthotic device of the present invention preferably includes a mechanism capable of optionally adjusting the fastening force during wearing in the fabric band A1 and/or the fabric band A2. By arbitrarily adjusting the fastening force, when the wearer puts on the lower-limb orthotic device of the present invention and then operates, the fabric band A1 or/and the fabric band A2 are not displaced from a predetermined portion, detached, or the like, in the fabric band A1, at least a part of the ankle portion including the malleolus and/or at least a part of the knee portion can be stably covered and gripped, in the fabric band A2, the metatarsal bone or cuboid bone of the foot portion can be stably covered and gripped, and in the fabric bands A1 and A2, the fabric band B suitably acts as a support exhibiting an orthotic force. The above adjustment mechanism is not particularly limited, and a belt, a hook, a hook-and-loop fastener, a button, a buckle, a loop, and the like can be appropriately selected according to the application.

The lower-limb orthotic device of the present invention preferably includes a mechanism that is openable and closable during wearing. The mechanism that is openable and closable during wearing is a mechanism that is openable and closable to expand an opening when the lower-limb orthotic device is worn and removed, or can open and close a portion that is not opened to facilitate wearing and removing. A portion with the openable and closable mechanism provided is not particularly limited as long as it is a portion where the orthotic function is not impaired and wearing and removing are facilitated, and the mechanism can also be provided in any one of the fabric band A1, the fabric band A2, and the fabric band B and in a connecting portion thereof. The number of portions with the openable and closable mechanism provided may be one or more.

In particular, the fabric band B preferably includes a length adjustment mechanism. As the length adjustment mechanism, a mechanism capable of pulling the fabric band B in the upper direction from the vicinity of the connecting portion between the fabric band A2 and the fabric band B is preferred. As a result, by adjusting the length of the fabric band B, it is possible to set an orthotic force according to a symptom of the wearer such as talipes equinovarus. In the mechanism for adjusting the length, by adopting the mechanism for pulling the fabric band B in the upper direction, a hemiplegic patient can easily wear the fabric band B with one hand. Thereby, it is possible to provide an orthotic device that can be continuously used.

As a general symptom of a hemiplegic patient, one hand and one foot may be in a paralyzed state on one side of the body, and a pulling action among actions of one hand that is not in a paralyzed state may be most easily performed. On the premise thereof, during wearing, particularly, when the lower-limb orthotic device of the present invention is worn while the position of the abnormal foot such as talipes equinovarus is returned to a normal position, the orthotic force can also be adjusted at the same time by adjusting the length of the fabric band B while pulling the fabric band B in the upper direction, which is excellent.

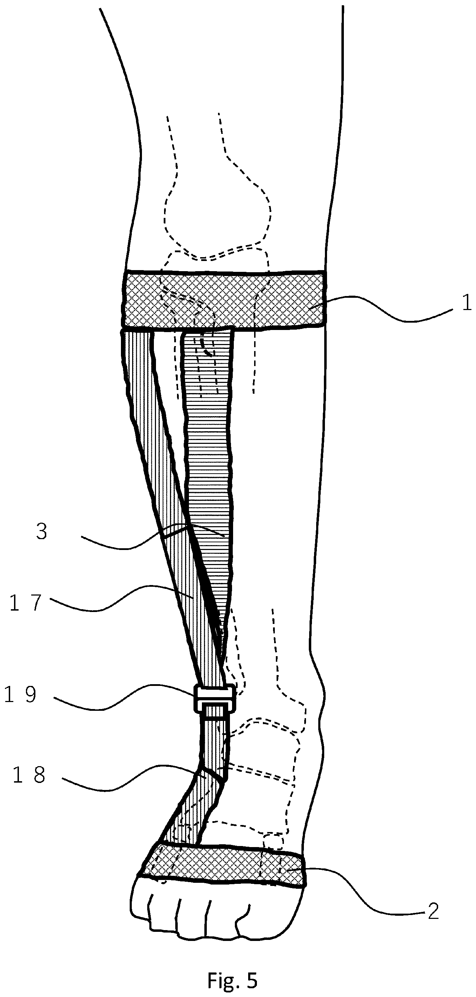

Specific examples of the length adjustment mechanism include a mechanism (mechanism 1) in which, when the fabric band B and the fabric band A2 are connected, a hole is provided on the fabric band side, the fabric band B is passed through the hole and folded back, and the fabric band B is pulled to be fastened, thereby adjusting the length, and a mechanism (mechanism 2) in which the fabric band B is configured by two or more members as illustrated in Fig. 5, and the fabric band B on the connection side with the fabric band A2 is pulled from the connecting portion to be able to be connected with the fabric band B on the connection side with the fabric band A1. Examples of the mechanism 1 include a method in which a hole, a loop, or the like provided in the fabric band A2 is used as the connecting portion, the fabric band B is passed through the hole, the loop, or the like and folded back, and the end of the fabric band B is fixed to the fabric band B with a hook-and-loop fastener or other fixing tools. Examples of the mechanism 2 include a mechanism in which the fabric band B is configured by two members b1 and b2, the member b1 is connected to the fabric band A1, the member b2 is connected to the fabric band A2, and the member b2 is pulled to fix the members b1 and b2 when both the members b1 and b2 are connected, thereby adjusting the length. Examples of a method of fixing b1 and b2 include a method of passing b1 and b2 through a loop and folding b1 and b2 back, and fixing b1 and b2 with a hook-and-loop fastener, and a method of connecting b1 and b2 with a fixing tool such as a ladder lock or a buckle. In the case of using the mechanism 2, the connecting portion is preferably provided on the instep or at a portion that is equal to or lower than at least the height of the ankle. Needless to say, the length adjustment mechanism such as the mechanisms 1 and 2 may also function as an openable and closable mechanism.

The mechanism that is openable and closable during wearing is included, whereby a wearer with strong deformation of the foot portion, an aged wearer, and the like can wear and remove the orthotic device by themselves, the wearing and removing work is reduced for the caregiver, and use of the orthotic device is not avoided and can be continuous. When the lower-limb orthotic device of the present invention can be continuously used, this case is more effective for function recovery and daily operation improvement. The openable and closable mechanism is not particularly limited, and a fastener such as a zip fastener or a hook-and-loop fastener, a button such as a snap button or a tack button, and the like can be appropriately selected according to the application. For example, by providing an opening/closing port with a fastener on the side surface of the lower leg, wearing and removing can be more easily performed.

The lower-limb orthotic device of the present invention may be used by being integrally formed with a leg wear covering at least from the knee to the foot portion as long as desired properties such as an orthotic force and detachability are satisfied in the fabric band, or the fabric band and the leg wear may be partially connected, but it is more preferable that the fabric band and the leg wear are connected and integrally formed. In the fabric band A1, at least a part of a portion covering the front portion of the knee portion such as the kneecap or the front portion of the ankle portion is preferably connected to the leg wear. In the fabric band B, at least a part of a portion covering the lower leg including the tibia is preferably connected to the leg wear from the viewpoint of detachability. Of course, both of these may be performed.

When the fabric band and the leg wear are integrally formed by being connected to each other, it is easy to dispose each fabric band in a desired manner during wearing, and it is possible to wear the fabric band in the same manner as general clothing without requiring time and effort for the disposition. As a result, the burden on the wearer and the caregiver can be reduced, and the lower-limb orthotic device of the present invention can be continuously used. As a result, it is more effective for function recovery and daily operation improvement of the wearer. The connection method is not particularly limited, and sewing, adhesion, and the like can be appropriately selected according to the application. From the viewpoint of continuous use, the lower limb orthosis of the present invention is preferably a brace that does not include a metal or hard resin frame, a monocoque, or the like, that is, a so-called soft brace.

## EXAMPLES

Hereinafter, examples of the present invention will be described together with comparative examples.

Methods of measuring various properties in the present examples are as follows.

(1) Fineness and number of filaments

The fineness was measured in compliance with fineness based on corrected mass (method A) in JIS L 1013: 2010 8.3.1. The number of filaments was measured on the basis of JIS L 1013: 2010 8.4.

(2) Extension ratio at load of 100 N/5 cm

Five test pieces each having a size of 50 mm × 300 mm were taken in each of the warp and weft directions of the woven fabric used for the fabric band. Using a constant-speed extension-type tensile tester with an automatic recording device, the grip interval was set to 200 mm, and the slack and tension of the test piece were removed and then the test piece was fixed to the grip. The test piece was stretched at a tensile speed of 200 mm/min until a load of 150 N was reached, the displacement at 100 N was measured on the basis of a load deformation curve obtained by performing the measurement, an extension ratio L (%) was determined by the following equation, and the extension ratio was represented as an average of five test pieces.

$$\text{Extension ratio L (\%)} = (L1/L) \times 100$$

L: grip interval (mm)
L1: displacement at an extension to 100 N (mm)

(3) Extension ratio at load of 4 N/cm (20 N/5 cm)

Five test pieces each having a size of 50 mm × 300 mm were taken in each of the warp and weft directions of the woven fabric used for the fabric band. Using a constant-speed extension-type tensile tester with an automatic recording device, the grip interval was set to 200 mm, and the slack and tension of the test piece were removed and then the test piece was fixed to the grip. The test piece was stretched at a tensile speed of 200 mm/min to 150 N, the displacement at 20 N was measured on the basis of a load deformation curve obtained by performing the measurement, an extension ratio LA (%) was determined by the following equation, and the extension ratio was represented as an average of five test pieces.

$$\text{Extension ratio LA (\%)} = (La1/La) \times 100$$

La: grip interval (mm)
La1: displacement at an extension to 20 N (mm)

(4) Extension change rate of fabric band B in longitudinal direction:

Five test pieces each having a size of 50 mm × 300 mm were taken in each of the warp and weft directions of the woven fabric used for the fabric band B. Using a constant-speed extension-type tensile tester with an automatic recording device, the test piece was marked with a grip interval of 200 m (Lb), and the slack and tension of the test pieces were removed and then the test pieces was fixed to the grip.

A cycle test is performed in which the operation of increasing the load of the test piece from 1 N to 50 N and then decreasing the load from 50 N to 1 N is defined as one set, this test is repeated 1000 times, and the cycle speed is set to 1 time/1 sec. A grip interval (Lb1) at the time of extension to 50 N in the first set of the above operation and a grip interval (Lb2) at the time of extension to 50 N in the 1000-th set were measured, and an extension change rate LB (%) was determined by the following equation and expressed as an average of five test pieces.

$$\text{Extension change rate LB (\%)} = [(Lb2 - Lb1)/Lb] \times 100$$

(5) Extension ratio of fabric band B at load of 10 N/cm (50 N/5 cm) in longitudinal direction

Five test pieces each having a size of 50 mm × 300 mm were taken in each of the warp and weft directions of the woven fabric used for the fabric band B. Using a constant-speed extension-type tensile tester with an automatic recording device, the grip interval was set to 200 mm, and the slack and tension of the test piece were removed and then the test piece was fixed to the grip. The test piece was stretched at a tensile speed of 200 mm/min to 150 N, the displacement at 50 N was measured on the basis of a load deformation curve obtained by performing the measurement, an extension ratio LC (%) was determined by the following equation, and the extension ratio was represented as an average of five test pieces.

$$\text{Extension ratio LC (\%)} = [(Lc1 - Lc)/Lc] \times 100$$

Lc: grip interval (mm)
Lc1: displacement at an extension to 50 N (mm)

(6) Extension recovery rate

Five test pieces each having a size of 50 mm × 300 mm were taken in each of the warp and weft directions. Using a constant-speed extension-type tensile tester with an automatic recording device, the test piece was marked with a grip interval of 200 m (Ld), and the slack and tension of the test piece were removed and then the test piece was fixed to the grip. The test piece was extended to 100 N at a tensile speed of 200 mm/min, left for 1 minute, then returned to the original position at the same speed, and left for 3 minutes. This operation was repeated 50 times, then the load was removed, and the test piece was left for 3 minutes. Then, the slack and tension of the test piece were removed, and the length (Ld1) between the marks was measured. The extension recovery rate LD (%) was determined by the following formula, and the average value for five test pieces was taken.

$$\text{Extension ratio LD (\%)} = [Ld - (Ld1 - Ld)/Ld] \times 100$$

(7) Method for measuring interior angle between fabric band A1 and fabric band B:

When the produced lower-limb orthotic device was worn on one foot requiring correction, an angle of 90° or less among angles formed by center lines by both end tangents of the fabric band A1 and the fabric band B in the longitudinal direction was measured.

(8) Method for measuring interior angle between fabric band B and fabric band C:

When the produced lower-limb orthotic device was worn on one foot requiring correction, an angle of 90° or less among angles formed by center lines by both end tangents of the fabric band B and the fabric band C in the longitudinal direction was measured.

(9) Wearing evaluation:

The produced lower-limb orthotic device was worn on one foot requiring correction, and in a state of walking for 1 hour, five items of dorsiflexion correction, inversion correction, orthotic force, compression feeling, and rubbing were evaluated. The dorsiflexion correction and the inversion correction were evaluated by observing the walking state of the wearer, the orthotic force, the compression feeling, and the rubbing were evaluated by sensory evaluation performed by the wearer, and these were evaluated in three grades of ⊙, ○, and ×.

A. Dorsiflexion correction

⊙: In swing phase of walking, the wearer walks so that the foot is dorsiflexed and the heel touches the ground before the tip of the foot when foot lands.
O: In swing phase of walking, the wearer walks so that the foot is dorsiflexed and the heel and the tip of the foot touch the ground at the same time when foot lands.
×: In swing phase of walking, the wearer walks so that the foot cannot be dorsiflexed and the tip of the foot touches the ground when foot lands.

B. Inversion correction

⊙: there is no inversion of the foot during walking, and the wearer walks so that the outer edge and the inner edge of the sole touch the ground when foot lands.
O: there is slightly inversion of the foot during walking, and the wearer walks so that the outer edge and the inner edge of the sole touch the ground when foot lands.
×: inversion of the foot during walking cannot be corrected, the wearer walks so that the outer edge of the sole touches the ground when foot lands, and walking becomes unstable.

C. Orthotic force

⊙: the orthotic state is maintained, and the fabric band is slightly tracked when force is applied.
○: the orthotic state is strongly maintained, and the fabric band does not change when force is applied.
×: retention in the orthotic state is weak, and the fabric band is tracked when force is applied.

D. Compression feeling

⊙: there is a feeling of strangeness in a portion covered by the fabric band during walking; however, it is not noticeable.
O: there is a compression feeling in a portion covered by the fabric band during walking; however, there is no pain.
×: there is a strong compression feeling in a portion covered by the fabric band, and there is pain.

E. Rubbing

⊙: there is no feeling of pain around the fabric band during walking.
○: there is no feeling of pain around the fabric band B during walking; however, there is a feeling of strangeness like rubbing.
×: there is a feeling of pain by rubbing with the skin around the fabric band during walking.

(10) Weave density

The woven density was measured on the basis of JIS L 1096: 2010 8.6.1 (method A). The sample was placed on a flat table, unnatural wrinkles and tension were removed, the number of warps and wefts existing at an interval of 2.54 cm (1 inch) was counted at five different locations, and the average value of each number was calculated.

(11) Crimp ratio

The crimp ratios for the warp and weft taken from the woven fabric were measured in accordance with JIS L1096: 2010 8.7 (method B). The measurement was performed for 20 yarns, and the average value thereof was taken.

(12) Wearing pressure

The wearing pressure of each of the fabric bands A1 and A2 with respect to the human body during wearing the lower-limb orthotic device was measured by an air-pack type wearing pressure measuring device AMI3037-10 manufactured by AMI Techno CO., LTD.
The wearing pressure of the fabric band A1 was measured at the portion of the knee sole, and the wearing pressure of the fabric band A2 was measured at the instep including the metatarsal bone.

[Example 1]

In the leg wear, there was used a tricot fabric obtained by interknitting two types of fibers, a polyester fiber of 54 decitex and a polyurethane fiber of 156 decitex as elastic fibers.
In the woven fabric used for the fabric band, using 167 decitex polyester (polyethylene terephthalate) fibers of inelastic yarns as the warp and 700 decitex polyester elastomer "Hytrel" (registered trademark) monofilament of elastic yarns as the weft, a plain woven fabric having a warp density of 15.35 yarns/cm (39 yarns/inch (2.54 cm)) and a weft density of 16.93 yarns/cm (43 yarns/inch (2.54 cm)) was produced. The obtained woven fabric was heat-treated at a temperature of 180°C for 2 minutes to provide a woven fabric having a warp density of 17.72 yarns/cm (45 yarns/inch (2.54 cm)) and a weft density of 18.11 yarns/cm (46 yarns/inch (2.54 cm)). The crimp ratios of the warp and the weft were 20% and 3%, respectively.
The extension ratio of the obtained woven fabric at a load of 20 N/cm (100 N/5 cm) was 2% in the warp direction and 8% in the weft direction, the extension ratio at a load of 4 N/cm (20 N/5 cm) was 1% in the warp direction and 2% in the weft direction, and the extension recovery rate after 50 times of extension at a load of 20 N/cm (100 N/5 cm) was 92% in the warp direction and 93% in the weft direction. The weft direction of the obtained woven fabric was set as the longitudinal direction of the fabric band, the extension ratio of the fabric band at a load of 10 N/cm (50 N/5 cm) in the longitudinal direction was 3%, and the extension change rate of the fabric band in the longitudinal direction was 12% after extension and restoration at the load were repeated 1000 times.
The leg wear has a sock shape covering from the foot portion to the knee portion of one foot, the fabric band A1 covers the knee portion including the popliteal fossa so that a circumferential direction of the knee and the weft direction of the obtained woven fabric are aligned, and the fabric band A2 goes around to cover the foot portion including the metatarsal bone. At this time, the width direction of the foot and the weft direction of the obtained woven fabric were aligned. One end of the fabric band B was connected to the fabric band A2 such that the weft direction of the woven fabric was set as the longitudinal direction. This connecting portion was formed in the vicinity of the foot portion including the cuboid bone on the fifth metatarsal bone side of the fabric band A2, the fabric band B was extended to the fabric band A1 from the outside to the

inside through the front portion of the lower leg portion including the tibia to cover the foot portion including the cuboid bone, and the fabric band B was connected to the fabric band A1 by forming the interior angle between the fabric band A1 and the fabric band B at 45° when the lower-limb orthotic device was worn. The wearing pressure of each of the fabric bands A1 and A2 with respect to the human body was adjusted to 15 hPa. The fabric band B was worn by the wearer, and was adjusted to a length that did not apply excessive stress in a state where the heel touched the ground in the standing state.

It became possible to adjust the fastening force to a desired fastening force by connecting a part of a portion covering the knee portion including the kneecap in the fabric band A1 and a part of a portion covering the front portion of the lower leg including the tibia in the fabric band B to the leg wear by sewing with a sewing machine, by integrally connecting the fabric band A2 to the fabric band B by sewing with a sewing machine, and by circling the end of the fabric band A1 in the circumferential direction (longitudinal direction) and the fabric band A2 lined with a hook-and-loop fastener to cover the foot portion including the metatarsal bone B and bonding the contact surface with the lower leg by the hook-and-loop fastener.

The extension ratio of the fabric band A2 lined with a hook-and-loop fastener at a load of 20 N/cm (100 N/5 cm) was 1.5% in the width direction and 6% in the longitudinal direction, the extension ratio at a load of 4 N/cm (20 N/5 cm) was 0.5% in the width direction and 1.5% in the longitudinal direction, and the extension recovery rate after 50 times of extension at a load of 20 N/cm (100 N/5 cm) was 94% in the width direction and 94% in the longitudinal direction.

When a wearing test was performed on the prepared lower-limb orthotic device, the dorsiflexion correction was evaluated as ⊙, the inversion correction was evaluated as ⊙, the orthotic force was evaluated as ⊙, the compression feeling was evaluated as ⊙, and the rubbing was evaluated as ⊙. As the lower-limb orthotic device, a foot requiring correction was corrected to a normal position, and there was no inversion of an ankle joint in swing phase of walking. When the foot touches the ground, the wearer was able to walk stably as the heel touched the ground first, and the wearer swung backward and forward with the tip of the foot. Both the orthotic force and the holding force necessary for maintaining the orthotic state of the foot and the trackability to friction, fluctuation, compression and the like occurring during operation between the lower limb and the lower-limb orthotic device were achieved, and the recovery at the time of deformation was also sufficient. Since this lower-limb orthotic device can be continuously used, the lower-limb orthotic device exhibits excellent performance. When the wearer wears this lower-limb orthotic device at home, the wearer can easily wear the lower-limb orthotic device without damaging the floor. When the wearer put on this lower-limb orthotic device and put on the shoe, the shoe was able to be fitted to the size of the foot. The sock shape facilitated the wearing and removing, and facilitated position adjustment of the fabric band A1 and the fabric band A2.

[Example 2]

A sock-type lower-limb orthotic device was used in the same manner as in Example 1, except that the interior angle between the fabric band A1 and the fabric band B when the lower-limb orthotic device was worn was adjusted to 65°.

When a wearing test was performed on the prepared lower-limb orthotic device, the dorsiflexion correction was evaluated as ⊙, the inversion correction was evaluated as ⊙, the orthotic force was evaluated as ⊙, the compression feeling was evaluated as ⊙, and the rubbing was evaluated as ⊙. As the lower-limb orthotic device, a foot requiring correction was corrected to a normal position, and there was no inversion of an ankle joint in swing phase of walking. When the foot touches the ground, the wearer was able to walk stably as the heel touched the ground first, and the wearer swung backward and forward with the tip of the foot. Both the orthotic force and the holding force necessary for maintaining the orthotic state of the foot and the trackability to friction, fluctuation, compression and the like occurring during operation between the lower limb and the lower-limb orthotic device were achieved, and the recovery at the time of deformation was also sufficient. Since this lower-limb orthotic device can be continuously used, the lower-limb orthotic device exhibits excellent performance. When the wearer wears this lower-limb orthotic device at home, the wearer can easily wear the lower-limb orthotic device without damaging the floor. When the wearer put on this lower-limb orthotic device and put on the shoe, the shoe was able to be fitted to the size of the foot. The sock shape facilitated the wearing and removing, and facilitated position adjustment of the fabric band A1 and the fabric band A2.

[Example 3]

A sock-type lower-limb orthotic device was used in the same manner as in Example 1, except that the interior angle between the fabric band A1 and the fabric band B when the lower-limb orthotic device was worn was adjusted to 85°.

When a wearing test was performed on the prepared lower-limb orthotic device, the dorsiflexion correction was evaluated as ⊙, the inversion correction was evaluated asO, the orthotic force was evaluated as ⊙, the compression feeling was evaluated as ⊙, and the rubbing was evaluated as ⊙. As the lower-limb orthotic device, it was possible to correct the foot requiring correction to the normal position. In swing phase of walking, there is slight inversion of the ankle joint, but when the foot touched the ground, the wearer was able to walk stably as the heel touched the ground first, and the wearer swung backward and forward with the tip of the foot. Both the orthotic force and the holding force necessary for maintaining the orthotic state of the foot and the trackability to friction, fluctuation, compression and the like occurring

during operation between the lower limb and the lower-limb orthotic device were achieved, and the recovery at the time of deformation was also sufficient. Since this lower-limb orthotic device can be continuously used, the lower-limb orthotic device exhibits excellent performance. When the wearer wears this lower-limb orthotic device at home, the wearer can easily wear the lower-limb orthotic device without damaging the floor. When the wearer put on this lower-limb orthotic device and put on the shoe, the shoe was able to be fitted to the size of the foot. The sock shape facilitated the wearing and removing, and facilitated position adjustment of the fabric band A1 and the fabric band A2.

[Example 4]

A sock-type lower-limb orthotic device was used in the same manner as in Example 1, except that the warp direction of the obtained woven fabric was set as the longitudinal direction of the fabric band. The extension ratio of the woven fabric at a load of 50 N/5 cm in the longitudinal direction was 1.7%, and the extension change rate of the woven fabric in the longitudinal direction was 9% after extension and restoration at the load were repeated 1000 times.

When a wearing test was performed on the prepared lower-limb orthotic device, the dorsiflexion correction was evaluated as ⊙, the inversion correction was evaluated as ⊙, the orthotic force was evaluated as ⊙, the compression feeling was evaluated as ⊙, and the rubbing was evaluated as ⊙. As the lower-limb orthotic device, a foot requiring correction was corrected to a normal position, and there was no inversion of an ankle joint in swing phase of walking. When the foot touches the ground, the wearer was able to walk stably as the heel touched the ground first, and the wearer swung backward and forward with the tip of the foot. Both the orthotic force and the holding force necessary for maintaining the orthotic state of the foot and the trackability to friction, fluctuation, compression and the like occurring during operation between the lower limb and the lower-limb orthotic device were achieved, and the recovery at the time of deformation was also sufficient. Since this lower-limb orthotic device can be continuously used, the lower-limb orthotic device exhibits excellent performance. When the wearer wears this lower-limb orthotic device at home, the wearer can easily wear the lower-limb orthotic device without damaging the floor. When the wearer put on this lower-limb orthotic device and put on the shoe, the shoe was able to be fitted to the size of the foot. The sock shape facilitated the wearing and removing, and facilitated position adjustment of the fabric band A1 and the fabric band A2.

[Example 5]

The fabric band C was added to the lower-limb orthotic device of Example 3, and one end of the fabric band C in the longitudinal direction was connected to the fabric band A2. The connecting place was near the foot portion including the first metatarsal bone of the fabric band A2. The fabric band C was disposed to cover the foot portion including the first metatarsal bone, cover from the plantar medial side to the lateral side from the lateral side of the heel to the medial side of the ankle portion including the Achilles tendon, and cover from the lower leg including tibia to the knee portion. A part of a portion covering the lower leg including the tibia of the fabric band B intersected with the fabric band C. The intersecting portions were connected by a sewing machine, and the interior angle between the fabric band B and the fabric band C when the lower-limb orthotic device was worn was formed to 45° and the fabric band B and the fabric band C was connected to the fabric band A1. Except for the above, a sock-type lower-limb orthotic device was used in the same manner as in Example 1. The extension ratio of the fabric band at a load of 10 N/cm (50 N/5 cm) in the longitudinal direction was 1.7%, and the extension change rate of the fabric band in the longitudinal direction was 9% after extension and restoration at the load were repeated 1000 times.

When a wearing test was performed on the prepared lower-limb orthotic device, the dorsiflexion correction was evaluated as ⊙, the inversion correction was evaluated as ⊙, the orthotic force was evaluated as ⊙, the compression feeling was evaluated as ⊙, and the rubbing was evaluated as ⊙. As the lower-limb orthotic device, a foot requiring correction was corrected to a normal position, and there was no inversion of an ankle joint in swing phase of walking. When the foot touches the ground, the wearer was able to walk stably as the heel touched the ground first, and the wearer swung backward and forward with the tip of the foot. Both the orthotic force and the holding force necessary for maintaining the orthotic state of the foot and the trackability to friction, fluctuation, compression and the like occurring during operation between the lower limb and the lower-limb orthotic device were achieved, and the recovery at the time of deformation was also sufficient. Since this lower-limb orthotic device can be continuously used, the lower-limb orthotic device exhibits excellent performance. When this lower-limb orthotic device is worn at home, the wearer can easily wear the lower-limb orthotic device without damaging the floor. When the wearer put on this lower-limb orthotic device and put on the shoe, the shoe was able to be fitted to the size of the foot. The sock shape facilitated the wearing and removing, and facilitated position adjustment of the fabric band A1 and the fabric band A2. By adding the fabric band C, the trunk forward tilt due to the functional deterioration of the tibialis anterior was improved during walking of the wearer, and the wearer could walk more stably.

[Example 6]

A lower-limb orthotic device was produced by the same method as in Example 1, except that a mechanism was employed in which the fabric band B of the lower-limb orthotic device are configured by two members b1 (elastic fabric band B (b1) 17) and b2 (elastic fabric band B (b2) 18) as illustrated in Fig. 5, the member b1 is connected to the fabric band A1, the member b2 is connected to the fabric band A2, the member b2 is pulled to be fixed to the member b1 when both the members b1 and b2 are connected, thereby adjusting the length, and a method of passing a member through a loop 19 and folding the member back, and fixing the member with a hook-and-loop fastener was employed as a method of fixing b1 and b2. The fabric band B was worn by the wearer and was then adjusted to a length that did not apply excessive stress in a state where the heel touched the ground in the standing state, and the wearing test was performed.

When a wearing test was performed on the prepared lower-limb orthotic device, the dorsiflexion correction was evaluated as ⊙, the inversion correction was evaluated as ⊙, the orthotic force was evaluated as ⊙, the compression feeling was evaluated as ⊙, and the rubbing was evaluated as ⊙. As the lower-limb orthotic device, a foot requiring correction was corrected to a normal position, and there was no inversion of an ankle joint in swing phase of walking. When the foot touches the ground, the wearer was able to walk stably as the heel touched the ground first, and the wearer swung backward and forward with the tip of the foot. Both the orthotic force and the holding force necessary for maintaining the orthotic state of the foot and the trackability to friction, fluctuation, compression and the like occurring during operation between the lower limb and the lower-limb orthotic device were achieved, and the recovery at the time of deformation was also sufficient. Since this lower-limb orthotic device can be continuously used, the lower-limb orthotic device exhibits excellent performance. When this lower-limb orthotic device is worn at home, the wearer can easily wear the lower-limb orthotic device without damaging the floor. When the wearer put on this lower-limb orthotic device and put on the shoe, the shoe was able to be fitted to the size of the foot. The sock shape facilitated the wearing and removing, and facilitated position adjustment of the fabric band A1 and the fabric band A2. By the adjusting the length of the fabric band B, it was possible to set an orthotic force according to a symptom of the wearer such as talipes equinovarus, and a hemiplegic patient more easily wore the fabric band B with one hand.

[Comparative Example 1]

A sock-type lower-limb orthotic device was used in the same manner as in Example 1, except that the interior angle between the fabric band A1 and the fabric band B when the lower-limb orthotic device was worn was adjusted to 20°.

When a wearing test was performed on the prepared lower-limb orthotic device, the dorsiflexion correction was evaluated as ×, the inversion correction was evaluated as ×, the orthotic force was evaluated as ⊙, the compression feeling was evaluated as ⊙, and the rubbing was evaluated as ⊙. As the lower-limb orthotic device, the interior angle between the fabric band A1 and the fabric band B was low, and it was not possible to correct the foot requiring correction to a normal position. In swing phase of walking, the ankle joint plantarflexed inward, the tip of the foot touched the ground first when the foot touched the ground, and when the tip of the foot tried to kick backward, the balance of the lower limb was lost, so that the wearer was not able to walk stably. Therefore, functions necessary for improving the deformation of the foot and the walking function were not recognized, which was inappropriate.

[Comparative Example 2]

In the leg wear, there was used a tricot fabric obtained by interknitting two types of fibers, a polyester (polyethylene terephthalate) fiber of 54 decitex and a polyurethane fiber of 156 decitex as elastic fibers.

In the woven fabric used for the fabric band, using doubled and twisted yarn obtained by combining 80 decitex polyester (polyethylene terephthalate) fibers and 80 decitex polyurethane fibers "LYCRA" (registered trademark) of elastic yarns as both the warp and the weft, a plain woven fabric having a warp density of 16.14 yarns/cm (41 yarns/inch (2.54 cm)) and a weft density of 16.93 yarns/cm (43 yarns/inch (2.54 cm)) was produced. The obtained woven fabric was heat-treated at a temperature of 130°C for 2 minutes to provide a woven fabric structure having a warp density of 17.72 yarns/cm (45 yarns/inch (2.54 cm)) and a weft density of 18.11 yarns/cm (46 yarns/inch (2.54 cm)). The crimp ratios of the warp and the weft were 5% and 3%, respectively. The extension ratio of the obtained woven fabric at a load of 20 N/cm (100 N/5 cm) was 45% in the warp direction and 47% in the weft direction, the extension ratio at a load of 4 N/cm (20 N/5 cm) was 15% in the warp direction and 17% in the weft direction, and the extension recovery rate after 50 times of extension at a load of 20 N/cm (100 N/5 cm) was 70% in the warp direction and 72% in the weft direction. The weft direction of the obtained woven fabric was set as the longitudinal direction of the fabric band, the extension ratio of the fabric band at a load of 10 N/cm (50 N/5 cm) in the longitudinal direction was 35%, and the extension change rate of the fabric band in the longitudinal direction was 30% after extension and restoration at the load were repeated 1000 times.

The extension ratio of the fabric band A2 lined with a hook-and-loop fastener at a load of 20 N/cm (100 N/5 cm) was 39% in the width direction and 40% in the longitudinal direction, the extension ratio at a load of 4 N/cm (20 N/5 cm) was

12% in the width direction and 15% in the longitudinal direction, and the extension recovery rate after 50 times of extension at a load of 20 N/cm (100 N/5 cm) was 72% in the width direction and 75% in the longitudinal direction.

A sock-type lower-limb orthotic device was used in the same manner as in Comparative Example 1, except that the above-described woven fabric was used for the fabric band. When a wearing test was performed on the prepared lower-limb orthotic device, the dorsiflexion correction was evaluated as ×, the inversion correction was evaluated as ×, the orthotic force was evaluated as ×, the compression feeling was evaluated as ⊙, and the rubbing was evaluated as ⊙. As the lower-limb orthotic device, the interior angle between the fabric band A1 and the fabric band B was low, and it was not possible to correct the foot requiring correction to a normal position. In swing phase of walking, the ankle joint plantarflexed inward, the tip of the foot touched the ground first when the foot touched the ground, and when the tip of the foot tried to kick backward, the balance of the lower limb was lost, so that the wearer was not able to walk stably. Therefore, functions necessary for improving the deformation of the foot and the walking function were not recognized, which was inappropriate.

[Comparative Example 3]

In the leg wear, there was used a tricot fabric obtained by interweaving two types of fibers, a polyester (polyethylene terephthalate) fiber of 54 decitex and a polyurethane fiber "LYCRA" (registered trademark) of 156 decitex as elastic fibers.

In the woven fabric used for the fabric band, using 167 decitex high-strength polyester (polyethylene terephthalate) fibers of inelastic yarns as both the warp and the weft, a plain woven fabric having a warp density of 16.93 yarns/cm (43 yarns/inch (2.54 cm)) and a weft density of 17.72 yarns/cm (45 yarns/inch (2.54 cm)) was produced. The obtained woven fabric was heat-treated at a temperature of 130°C for 2 minutes to provide a woven fabric structure having a warp density of 17.72 yarns/cm (45 yarns/inch (2.54 cm)) and a weft density of 18.11 yarns/cm (46 yarns/inch (2.54 cm)). The crimp ratios of the warp and the weft were 2% and 3%, respectively. The extension ratio of the obtained woven fabric at a load of 20 N/cm (100 N/5 cm) was 0.5% in the warp direction and 0.6% in the weft direction, the extension ratio at a load of 4 N/cm (20 N/5 cm) was 0.05% in the warp direction and 0.07% in the weft direction, and the extension recovery rate after 50 times of extension at a load of 20 N/cm (100 N/5 cm) was 94% in the warp direction and 95% in the weft direction. The weft direction of the obtained woven fabric was set as the longitudinal direction of the fabric band, the extension ratio of the fabric band at a load of 10 N/cm (50 N/5 cm) in the longitudinal direction was 0.1%, and the extension change rate of the fabric band in the longitudinal direction was 10% after extension and restoration at the load were repeated 1000 times.

The extension ratio of the fabric band A2 lined with a hook-and-loop fastener at a load of 20 N/cm (100 N/5 cm) was 0.2% in the width direction and 0.4% in the longitudinal direction, the extension ratio at a load of 4 N/cm (20 N/5 cm) was 0.04% in the width direction and 0.05% in the longitudinal direction, and the extension recovery rate after 50 times of extension at a load of 20 N/cm (100 N/5 cm) was 95% in the width direction and 96% in the longitudinal direction.

A sock-type lower-limb orthotic device was used in the same manner as in Comparative Example 1, except that the above-described fabric band was used instead of the fabric band of Comparative Example 1. When a wearing test was performed on the prepared lower-limb orthotic device, the dorsiflexion correction was evaluated as ×, the inversion correction was evaluated as ×, the orthotic force was evaluated as O, the compression feeling was evaluated as O, and the rubbing was evaluated as ×. As the lower-limb orthotic device, the interior angle between the fabric band A1 and the fabric band B was low, and it was not possible to correct the foot requiring correction to a normal position. In swing phase of walking, the ankle joint plantarflexed inward, the tip of the foot touched the ground first when the foot touched the ground, and when the tip of the foot tried to kick backward, the balance of the lower limb was lost, so that the wearer was not able to walk stably. Therefore, in this lower-limb orthotic device, functions necessary for improving the deformation of the foot and the walking function were not recognized. Since the extension ratio of the fabric band B at a load of 10 N/cm (50 N/5 cm) in the longitudinal direction was excessively low and the fabric band B did not follow the motion of the foot during operation, the holding force and the orthotic force necessary for improving the deformation of the foot and the walking function became excessively strong, and there was pain against the friction occurring during operation between the foot and the lower-limb orthotic device. In addition, when polyester of inelastic yarns was deformed, the shape was not restored to the shape before deformation, and was insufficient for continuous use and thus unsuitable.

[Comparative Example 4]

The same sock-type lower-limb orthotic device as in Example 1 was used, except that a commercially available rubber band was used instead of the woven fabric used for the fabric bands A1, A2, and B, and the interior angle between the rubber band in place of the fabric band A1 and the rubber band in place of the fabric band B when the lower-limb orthotic device was worn was adjusted to 20°.

When a wearing test was performed on the prepared lower-limb orthotic device, the dorsiflexion correction was evaluated as ×, the inversion correction was evaluated as ×, the orthotic force was evaluated as ×, the compression

feeling was evaluated as ⊙, and the rubbing was evaluated as ⊙. As the lower-limb orthotic device, the extension ratio of the fabric band was high, and it was difficult to correct the foot requiring correction to a normal position. In swing phase of walking, the ankle joint plantarflexed inward, the tip of the foot touched the ground first when the foot touched the ground, and when the tip of the foot tried to kick backward, the balance of the lower limb was lost, so that the wearer was not able to walk stably. Therefore, functions necessary for improving the deformation of the foot and the walking function were not recognized, which was inappropriate.

[Comparative Example 5]

A sock-type lower-limb orthotic device was used in the same manner as in Example 1, except that polyester elastomer "Hytrel" (registered trademark) monofilament was used for the fabric band and the interior angle between the fabric band A1 and the fabric band B when the lower-limb orthotic device was worn was adjusted to 20°.

When a wearing test was performed on the prepared lower-limb orthotic device, the dorsiflexion correction was evaluated as ×, the inversion correction was evaluated as ×, the orthotic force was evaluated as ×, the compression feeling was evaluated as ⊙, and the rubbing was evaluated as ⊙. As the lower-limb orthotic device, the interior angle between the fabric band A1 and the fabric band B was low, and it was not possible to correct the foot requiring correction to a normal position. In swing phase of walking, the ankle joint plantarflexed inward, the tip of the foot touched the ground first when the foot touched the ground, and when the tip of the foot tried to kick backward, the balance of the lower limb was lost, so that the wearer was not able to walk stably. Therefore, functions necessary for improving the deformation of the foot and the walking function were not recognized, which was inappropriate.

[Comparative Example 6]

The same sock-type lower-limb orthotic device as in Example 2 was used, except that the position of the fabric band A1 was set to a position of the maximum diameter in the calf.

When a wearing test was performed on the prepared lower-limb orthotic device, the dorsiflexion correction was evaluated as ×, the inversion correction was evaluated as ×, the orthotic force was evaluated as ×, the compression feeling was evaluated as ⊙, and the rubbing was evaluated as ⊙. As the lower-limb orthotic device, the position of the fabric band A1 was pushed down during wearing, and did not function as a fulcrum of the orthotic force, and it was difficult to correct the foot requiring correction to a normal position. In swing phase of walking, the ankle joint plantarflexed inward, the tip of the foot touched the ground first when the foot touched the ground, and when the tip of the foot tried to kick backward, the balance of the lower limb was lost, so that the wearer was not able to walk stably. Therefore, functions necessary for improving the deformation of the foot and the walking function were not recognized, which was inappropriate.

[Comparative Example 7]

The same sock-type lower-limb orthotic device as in Example 1 was used, except that a commercially available rubber band was used instead of the woven fabric used for the fabric bands A1, A2, and B.

When a wearing test was performed on the prepared lower-limb orthotic device, the dorsiflexion correction was evaluated as ×, the inversion correction was evaluated as ×, the orthotic force was evaluated as ×, the compression feeling was evaluated as ⊙, and the rubbing was evaluated as ⊙. As the lower-limb orthotic device, the extension ratio of the rubber band was high, and it was difficult to correct the foot requiring correction to the normal position. In swing phase of walking, the ankle joint plantarflexed inward, the tip of the foot touched the ground first when the foot touched the ground, and when the tip of the foot tried to kick backward, the balance of the lower limb was lost, so that the wearer was not able to walk stably. Therefore, functions necessary for improving the deformation of the foot and the walking function were not recognized, which was inappropriate.

[Table 1-1]

| | | | Example 1 | Example 2 | Example 3 | Example 4 | Example 5 | Example 6 |
|---|---|---|---|---|---|---|---|---|
| Physical properties of fabric band (Common to fabric bands A1, A2, B, and C) | Warp | | Polyester fiber | | | | | |
| | Weft | | "Hytrel" | | | | | |
| | Extension ratio at load of 20 N/5 cm | Warp direction | 1% | 1% | 1% | 1% | 1% | 1% |
| | | Weft direction | 2% | 2% | 2% | 2% | 2% | 2% |
| | Extension ratio at load of 100 N/5 cm | Warp direction | 2% | 2% | 2% | 2% | 2% | 2% |
| | | Weft direction | 8% | 8% | 8% | 8% | 8% | 8% |
| | Longitudinal direction of fabric band Extension ratio at load of 50 N/5 cm | | 3% | 3% | 3% | 1.7% | 3% | 3% |
| | After 1000 repetitions Extension change rate of fabric band in longitudinal direction | | 12% | 12% | 12% | 9% | 12% | 12% |
| Relationship of extension ratio of each fabric band at load of 100 N/5 cm | | | A1E=A2E=BE | | | | A1E=A2E=CE=BE | A1E=A2E=BE |
| Properties of lower-limb orthotic device during wearing | Interior angle between fabric band A1 and fabric band B | | 45° | 65° | 85° | 45° | 85° | 45° |
| | Interior angle between fabric band C and fabric band B | | - | - | - | - | 45° | - |
| Wearing evaluation item | Dorsiflexion correction | | ⊙ | ⊙ | ⊙ | ⊙ | ⊙ | ⊙ |
| | Inversion correction | | ⊙ | ⊙ | ○ | ⊙ | ⊙ | ⊙ |
| | Orthotic force | | ⊙ | ⊙ | ⊙ | ⊙ | ⊙ | ⊙ |
| | Compression feeling | | ⊙ | ⊙ | ⊙ | ⊙ | ⊙ | ⊙ |
| | Rubbing | | ⊙ | ⊙ | ⊙ | ⊙ | ⊙ | ⊙ |

[Table 1-2]

| | | | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|---|
| Physical properties of fabric band (Common to fabric bands A1, A2, B, and C) | Warp | | Polyester fiber | Doubled and twisted yarn of polyester fibers and polyurethane fibers | High-strength polyester fiber | Rubber band | "Hytrel" | Polyester fiber | Rubber band |
| | Weft | | "Hytrel" | | | | | "Hytrel" | |
| | Extension ratio at load of 20 N/5 cm | Warp direction | 1% | 15% | 0.05% | 30% | 10% | 1% | 30% |
| | | Weft direction | 2% | 17% | 0.07% | 10% | 10% | 2% | 10% |
| | Extension ratio at load of 100 N/5 cm | Warp direction | 2% | 45% | 0.5% | 90% | 40% | 2% | 90% |
| | | Weft direction | 8% | 47% | 0.6% | 25% | 40% | 8% | 25% |
| | Longitudinal direction of fabric band Extension ratio at load of 50 N/5 cm | | 3% | 35% | 0.1% | 50% | 25% | 3% | 50% |
| | After 1000 repetitions Extension change rate of fabric band in longitudinal direction | | 12% | 30% | 10% | 45% | 25% | 12% | 45% |
| Relationship of extension ratio of each fabric band at load of 100 N/5 cm | | | $A1_E = A2_E = B_E$ | | | | | | |
| Properties of lower-limb orthotic device during wearing | Interior angle between fabric band A1 and fabric band B | | 20° | 20° | 20° | 20° | 20° | 65° | 45° |
| | Interior angle between fabric band C and fabric band B | | - | - | - | - | - | - | - |
| Wearing evaluation item | Dorsiflexion correction | | × | × | × | × | × | × | × |
| | Inversion correction | | × | × | × | × | × | × | × |
| | Orthotic force | | ⊙ | × | ○ | × | × | × | × |
| | Compression feeling | | ⊙ | ⊙ | ○ | ⊙ | ⊙ | ⊙ | ⊙ |
| | Rubbing | | ⊙ | ⊙ | × | ⊙ | ⊙ | ⊙ | ⊙ |

DESCRIPTION OF REFERENCE SIGNS

1: Fabric band A1
2: Fabric band A2
3: Fabric band B
4: First metatarsal bone
5: Fifth metatarsal bone
6: Cuboid bone
7: Calcaneus
8: Talus
9: Tibia
10: Fibula
11: Lower leg
12: Interior angle between fabric band A1 and fabric band B
13: Fabric band C
14: Interior angle between fabric band B and fabric band C
15: Center line of fabric band B
16: Center line of fabric band C
17: Elastic fabric band B (b1)
18: Elastic fabric band B (b2)
19: loop

## Claims

**1.** A lower-limb orthotic device comprising:

a fabric band A1 (1) covering at least a part of an ankle portion including a malleolus and/or at least a part of a knee portion;
a fabric band A2(2) covering a foot portion including a metatarsal bone; and
at least one fabric band B (3),
wherein at least a part of the fabric band B (3) covers the foot portion including a fifth metatarsal bone (5) or a cuboid bone (6), the fabric band B (3) is disposed to be positioned from the foot portion including the fifth metatarsal bone (5) or the cuboid bone (6) to the ankle portion and/or the knee portion, one end of the fabric band B (3) in a longitudinal direction is connected to the fabric band A1 (1), the other end of the fabric band B (3) is connected to the fabric band A2 (2), an interior angle (12) between the fabric band A1 (1) and the fabric band B (3) when the lower-limb orthotic device is worn is 30° to 90°,
**characterized in that**:

at least one of the fabric bands (1, 2, 3) has an extension ratio at a load of 20 N/cm (100 N/5 cm) of 0.5% or more and 50% or less in a width direction and a longitudinal direction or in either one of the width direction and the longitudinal direction, and
wherein one or more kinds of thermoplastic elastomer fibers are contained as fibers constituting the fabric band A1 (1), the fabric band A2 (2), and the fabric band B (3).

**2.** The lower-limb orthotic device according to claim 1, wherein the fabric band B (3) includes a length adjustment mechanism.

**3.** The lower-limb orthotic device according to claim 1 or 2, wherein the one or more kinds of thermoplastic elastomer fibers contained as fibers constituting the fabric band A1, the fabric band A2, and the fabric band B include one or more kinds selected from polyurethane elastic fibers and thermoplastic polyester elastomer fibers.

**4.** The lower-limb orthotic device according to any one of claims 1 to 3, wherein polyester-based thermoplastic elastomer fibers are contained as fibers constituting the fabric band A1 (1), the fabric band A2 (2), and the fabric band B (3).

**5.** The lower-limb orthotic device according to any one of claims 1 to 4, further comprising one or more fabric bands C (13),
wherein at least a part of the fabric band C (13) is disposed to cover the foot portion including a first metatarsal bone, and the fabric band C (13) is formed by connecting one end of the fabric band C (13) in a longitudinal direction to the fabric band A1 (1) and the other end of the fabric band C (13) to the fabric band A2 (2) so as to be positioned from the foot portion including the first metatarsal bone (4) to the ankle portion and/or the knee portion.

**6.** The lower-limb orthotic device according to claim 5, wherein an interior angle (14) between the fabric band B (3) and the fabric band C (13) is 30° to 80°.

**7.** The lower-limb orthotic device according to claim 5 or 6, wherein one or more kinds of thermoplastic elastomer fibers are contained as fibers constituting the fabric band C (13).

**8.** The lower-limb orthotic device according to any one of claims 5 to 7, wherein polyester-based thermoplastic elastomer fibers are contained as fibers constituting the fabric band C (13).

**9.** The lower-limb orthotic device according to any one of claims 5 to 8, wherein, when the extension ratios of the fabric bands A1 (1), A2 (2), B (3), and C (13) at a load of 20 N/cm (100 N/5 cm) are represented as $A1_E$, $A2_E$, $B_E$, and $C_E$, respectively, $A1_E \geq C_E \geq B_E$ and $A2_E \geq C_2 \geq B_E$ are satisfied.

**10.** The lower-limb orthotic device according to any one of claims 1 to 9, wherein at least one of the fabric bands has an extension ratio at a load of 4 N/cm (20 N/5 cm) of 0.1% or more and 10% or less in the width direction and the longitudinal direction or in either one of the width direction and the longitudinal direction.

**11.** The lower-limb orthotic device according to any one of claims 1 to 10, wherein the lower-limb orthotic device is formed integrally with a leg wear that covers at least a knee to the foot portion, and includes a mechanism that is openable and

closable during wearing.

12. The lower-limb orthotic device according to any one of claims 1 to 11, wherein the lower-limb orthotic device is a brace that does not include a metal or hard resin frame, or a monocoque.

## Patentansprüche

1. Orthesevorrichtung für eine untere Gliedmaße, umfassend:

   eine Stoffbahn A1 (1), die zumindest einen Teil eines Fußgelenkabschnitts, welcher einen Fußknöchel und/oder zumindest einen Teil eines Knieabschnitts umfasst, bedeckt;
   eine Stoffbahn A2 (2), die einen Fußabschnitt, welcher einen Mittelfußknochen umfasst, bedeckt; und zumindest eine Stoffbahn B (3),
   wobei zumindest ein Teil der Stoffbahn B (3) den Fußabschnitt, welcher einen fünften Mittelfußknochen (5) oder ein Würfelbein (6) umfasst, bedeckt, wobei die Stoffbahn B (3) angeordnet ist, um von dem Fußabschnitt, welcher den fünften Mittelfußknochen (5) oder das Würfelbein (6) umfasst, bis zu dem Fußgelenkabschnitt und/oder dem Knieabschnitt platziert zu sein, wobei ein Ende der Stoffbahn B (3) in einer Längsrichtung mit der Stoffbahn A1 (1) verbunden ist, wobei das andere Ende der Stoffbahn B (3) mit der Stoffbahn A2 (2) verbunden ist, wobei ein Innenwinkel (12) zwischen der Stoffbahn A1 (1) und der Stoffbahn B (3), wenn die Orthesevorrichtung für eine untere Gliedmaße getragen wird, 30° bis 90° beträgt,
   **dadurch gekennzeichnet, dass**
   zumindest eine der Stoffbahnen (1, 2, 3) bei einer Last von 20 N/cm (100 N/5 cm) ein Ausdehnungsverhältnis von 0,5% oder mehr und 50 % oder weniger in eine Breitenrichtung und in eine Längsrichtung oder in einer aus der Breitenrichtung und der Längsrichtung aufweist, und
   wobei eine oder mehrere Arten von thermoplastischen Elastomerfasern als Fasern, welche die Stoffbahn A1 (1), die Stoffbahn A2 (2) und die Stoffbahn B (3) bilden, enthalten sind.

2. Orthesevorrichtung für eine untere Gliedmaße nach Anspruch 1, wobei die Stoffbahn B (3) einen Längenanpassungsmechanismus umfasst.

3. Orthesevorrichtung für eine untere Gliedmaße nach Anspruch 1 oder 2, wobei die eine oder mehreren Arten von thermoplastischen Elastomerfasern als Fasern, welche die Stoffbahn A1, die Stoffbahn A2 und die Stoffbahn B bilden, eine oder mehrere Arten ausgewählt aus elastischen Polyurethanfasern und thermoplastischen Polyesterelastomerfasern umfassen.

4. Orthesevorrichtung für eine untere Gliedmaße nach einem der Ansprüche 1 bis 3, wobei thermoplastische Elastomerfasern als Fasern, welche die Stoffbahn A1 (1), die Stoffbahn A2 (2) und die Stoffbahn B (3) bilden, enthalten sind.

5. Orthesevorrichtung für eine untere Gliedmaße nach einem der Ansprüche 1 bis 4, ferner umfassend eine oder mehrere Stoffbahnen C (13),
   wobei zumindest ein Teil der Stoffbahn C (13) angeordnet ist, um einen Fußabschnitt, welcher einen ersten Mittelfußknochen umfasst, zu bedecken, und die Stoffbahn C (13) durch Verbinden eines Endes der Stoffbahn C (13) in eine Längsrichtung mit der Stoffbahn A1 (1) und des anderen Endes der Stoffbahn C (13) mit der Stoffbahn A2 (2) ausgebildet ist, sodass sie von dem Fußabschnitt, welcher den ersten Mittelfußknochen (4) umfasst, bis zu dem Knöchelabschnitt und/oder dem Knieabschnitt platziert ist.

6. Orthesevorrichtung für eine untere Gliedmaße nach Anspruch 5, wobei ein Innenwinkel (14) zwischen der Stoffbahn B (3) und der Stoffbahn C (13) 30° bis 80° beträgt.

7. Orthesevorrichtung für eine untere Gliedmaße nach Anspruch 5 oder 6, wobei eine oder mehrere Arten von thermoplastischen Elastomerfasern als Fasern, welche die Stoffbahn C (13) bilden, enthalten sind.

8. Orthesevorrichtung für eine untere Gliedmaße nach einem der Ansprüche 5 bis 7, wobei thermoplastische Elastomerfasern auf Polyesterbasis als Fasern, welche die Stoffbahn C (13) bilden, enthalten sind.

9. Orthesevorrichtung für eine untere Gliedmaße nach einem der Ansprüche 5 bis 8, wobei die Ausdehnungsverhältnisse der Stoffbahnen A1 (1), A2 (2), B (3) und C (13) bei einer Last von 20 N/cm (100 N/5 cm) jeweils als $A1_E$, $A2_E$,

$B_E$ bzw. $C_E$ dargestellt sind, wobei $A1_E \geq C_E \geq B_E$ und $A2_E \geq B_E \geq B_E$ erfüllt ist.

**10.** Orthesevorrichtung für eine untere Gliedmaße nach einem der Ansprüche 1 bis 9, wobei zumindest eine der Stoffbahnen bei einer Last von 4 N/cm (20 N/5 cm) ein Ausdehnungsverhältnis von 0,1 % oder mehr und 10 % oder weniger in Breitenrichtung und in Längsrichtung oder in einer aus der Breitenrichtung und der Längsrichtung aufweist.

**11.** Orthesevorrichtung für eine untere Gliedmaße nach einem der Ansprüche 1 bis 10, wobei die Orthesevorrichtung für eine untere Gliedmaße einstückig verbunden mit einer Beinbekleidung, welche zumindest ein Knie bis zu dem Fußabschnitt abdeckt, ausgebildet ist und einen Mechanismus umfasst, der während des Tragens öffenbar und schließbar ist.

**12.** Orthesevorrichtung für eine untere Gliedmaße nach einem der Ansprüche 1 bis 11, wobei die Orthesevorrichtung für eine untere Gliedmaße eine Stützstruktur ist, welche kein(en) Metall- oder Hartharzrahmen oder einschaliges Teil umfasst.

## Revendications

**1.** Dispositif orthopédique de membre inférieur, comprenant :

une bande de tissu A1 (1) recouvrant au moins une partie d'une portion de cheville comprenant une malléole et/ou au moins une partie d'une portion de genou ;
une bande de tissu A2 (2) recouvrant une portion de pied comprenant un os métatarsien ; et
au moins une bande de tissu B (3),
dans lequel au moins une partie de la bande de tissu B (3) recouvre la portion de pied comprenant un cinquième os métatarsien (5) ou un os cuboïde (6), la bande de tissu B (3) est disposée pour être positionnée depuis la portion de pied comprenant le cinquième os métatarsien (5) ou l'os cuboïde (6) jusqu'à la portion de cheville et/ou la portion de genou, une première extrémité de la bande de tissu B (3) dans une direction longitudinale est reliée à la bande de tissu A1 (1), l'autre extrémité de la bande de tissu B (3) est reliée à la bande de tissu A2 (2), un angle intérieur (12) entre la bande de tissu A1 (1) et la bande de tissu B (3), lorsque le dispositif orthopédique de membre inférieur est porté, est de 30° à 90°,
**caractérisé en ce que** :

au moins une des bandes de tissu (1, 2, 3) présente un rapport d'extension à une charge de 20 N/cm (100 N/5 cm) de 0,5 % ou plus et de 50 % ou moins dans une direction de largeur et une direction longitudinale ou dans l'une ou l'autre de la direction de largeur et de la direction longitudinale, et
dans lequel un ou plusieurs types de fibres d'élastomère thermoplastique sont contenus en tant que fibres constituant la bande de tissu A1 (1), la bande de tissu A2 (2), et la bande de tissu B (3).

**2.** Dispositif orthopédique de membre inférieur selon la revendication 1, dans lequel la bande de tissu B (3) inclut un mécanisme d'ajustement de longueur.

**3.** Dispositif orthopédique de membre inférieur selon la revendication 1 ou 2, dans lequel les un ou plusieurs types de fibres d'élastomère thermoplastique contenues sous forme de fibres constituant la bande de tissu A1, la bande de tissu A2 et la bande de tissu B comprennent un ou plusieurs types choisis parmi des fibres élastiques de polyuréthane et des fibres d'élastomère de polyester thermoplastique.

**4.** Dispositif orthopédique de membre inférieur selon l'une quelconque des revendications 1 à 3, dans lequel des fibres d'élastomère thermoplastique à base de polyester sont contenues sous forme de fibres constituant la bande de tissu A1 (1), la bande de tissu A2 (2) et la bande de tissu B (3).

**5.** Dispositif orthopédique de membre inférieur selon l'une quelconque des revendications 1 à 4, comprenant en outre une ou plusieurs bandes de tissu C (13),
dans lequel au moins une partie de la bande de tissu C (13) est disposée pour couvrir la portion de pied comprenant un premier os métatarsien, et la bande de tissu C (13) est formée en reliant une première extrémité de la bande de tissu C (13) dans une direction longitudinale à la bande de tissu A1 (1) et l'autre extrémité de la bande de tissu C (13) à la bande de tissu A2 (2) de manière à être positionnée à partir de la portion de pied comprenant le premier os métatarsien

(4) à la portion de cheville et/ou à la portion de genou.

**6.** Dispositif orthopédique de membre inférieur selon la revendication 5, dans lequel un angle intérieur (14) entre la bande de tissu B (3) et la bande de tissu C (13) est de 30° à 80°.

**7.** Dispositif orthopédique de membre inférieur selon la revendication 5 ou 6, dans lequel un ou plusieurs types de fibres d'élastomère thermoplastique sont contenus en tant que fibres constituant la bande de tissu C (13).

**8.** Dispositif orthopédique de membre inférieur selon l'une quelconque des revendications 5 à 7, dans lequel des fibres d'élastomère thermoplastique à base de polyester sont contenues sous forme de fibres constituant la bande de tissu C (13).

**9.** Dispositif orthopédique de membre inférieur selon l'une quelconque des revendications 5 à 8, dans lequel, lorsque les rapports d'extension des bandes de tissu A1 (1), A2 (2), B (3) et C (13) à une charge de 20 N/cm (100 N/5 cm) sont représentés par $A1_E$, $A2_E$, $B_E$ et $C_E$, respectivement, $A1_E \geq C_E \geq B_E$ et $A2_E \geq C_E \geq B_E$ sont satisfaites.

**10.** Dispositif orthopédique de membre inférieur selon l'une quelconque des revendications 1 à 9, dans lequel au moins l'une des bandes de tissu présente un rapport d'extension à une charge de 4 N/cm (20 N/5 cm) de 0,1 % ou plus et de 10 % ou moins dans la direction de la largeur et la direction longitudinale ou dans l'une ou l'autre de la direction de la largeur et de la direction longitudinale.

**11.** Dispositif orthopédique de membre inférieur selon l'une quelconque des revendications 1 à 10, dans lequel le dispositif orthopédique de membre inférieur est formé d'un seul tenant avec un vêtement de jambe qui couvre au moins un genou jusqu'à la portion de pied, et comprend un mécanisme qui peut être ouvert et fermé pendant le port.

**12.** Dispositif orthopédique de membre inférieur selon l'une quelconque des revendications 1 à 11, dans lequel le dispositif orthopédique de membre inférieur est une attelle qui ne comprend pas de cadre en métal ou en résine dure, ou une monocoque.

9
1
1 2
1 0
1 1
3
3
3
9
8
3
7
6
4
5
2

Fig. 1

1
1 2
3

Fig. 2

9
1
1 2
1 0
1 1
1 3
3
1 3
3
3
9
1 3
8
3
7
1 4
4
6
5
2

Fig. 3

1 6
1 5
1 4


Fig. 4

1
3
1 7
1 9
1 8
2

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H9313553 B
- US 5257969 A
- US 2015320581 A1
- GB 2375962 A
- IE 20100556 A1
- US 5860423 A